# EUROPEAN PATENT SPECIFICATION

(11) **EP 1 155 683 B1**
(45) Date of publication and mention of the grant of the patent: **02.11.2006**
(21) Application number: 01203158.9
(22) Date of filing: 18.08.1995
(51) Int. Cl.: A61K 8/29, A61Q 5/00, A61Q 17/04

(54) **Oral composition with an improved teeth whitening effect**
Orale Formulierung zum verbesserten Bleichen von Zähnen
Composition orale pour un blanchiment des dents avec un effet accru

(30) Priority: 22.08.1994 EP 94306191
(43) Date of publication of application: 21.11.2001
(62) Divisional of application: 95930506.1
(73) Proprietor: UNILEVER N.V., 3013 AL Rotterdam (NL); UNILEVER PLC, London EC4P 4BQ (GB)
(72) Inventor: Thornthwaite, David William, Bebington, Wirral, Merseyside CH63 3JW (GB); JOINER, Andrew, Bebington, Wirral, Merseyside CH63 3JW (GB)
(74) Representative: Newbould, Frazer Anthony

(56) References cited:
- EP-A- 0 316 809
- EP-A- 0 325 289
- EP-A- 0 450 587
- EP-A- 0 508 623
- US-A- 3 988 433
- US-A- 5 246 620
- US-A- 5 266 587

## Description

The present invention relates to an oral composition with an improved teeth whitening effect. More particularly, it relates to an oral composition with an improved teeth whitening effect comprising a safe and effective amount of certain organic peroxyacids.

The use of peroxy compounds in oral care compostions has already been proposed in the prior art. Many peroxy compounds have been suggested for whitening/bleaching human teeth, and representative examples of such peroxy compounds are hydrogen peroxide, urea peroxide, organic peracids such as perphthalic acid, diperoxycarboxylic acids, 1,12-dodecanedioic peroxy acid, peroxy acetic acid and systems comprising a peroxy compound and a peroxy acid precursor which generate peroxy acetic acid in situ, such as sodium perborate and tetraacetylethylene diamine (TAED). The use of peroxy acetic acid is suggested in particular in e.g. EP-A-0545,594 (Colgate), which also sets out the various prior proposals, made in the art for several peroxy compounds as bleaching/whitening agent for human teeth.

We have now found that certain organic peroxy acids, which will be defined hereinafter, are either more stable than peroxy acetic acid or are more effective than peroxy acetic acid. These certain organic peroxy acids are selected from the group consisting of:
1) cationic peroxycarboxylic acids having the formula 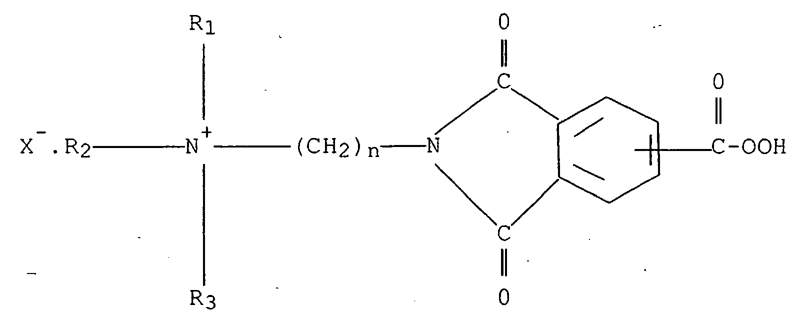
wherein R₁, R₂ and R₃ are each independently a C₁-C₇ alkyl group or C₁-C₇ substituted alkyl group, n is an integer of from 2 to 10 and X is a counter anion.

### 2 butyl imido peroxytrimellitic acid ("BIPTA")

The cationic peroxyacids of formula 1) are known per se, and have been described in EP-A-508,623. A preferred compound is trimethyl ammonium propenyl imidoperoxy-mellitic acid of formula 1), in which R₁, R₂ and R₃ are each methyl, n = 3 and X = methanesulphonate.

BIPTA is described in WO/91/09843;
Thus, the present invention relates to an oral composition with an improved teeth whitening effect, comprising a safe and effective amount of an organic peroxy acid as bleaching/whitening agent, characterised in that the organic peroxy acid is selected from the group consisting of peroxyacids 1) and 2) as defined above.

The amount of peroxyacids,used in the present invention, may vary from 0.01 to 99 % by weight of the oral composition, preferably from 0.1 to 30 % by weight, particularly preferably from 0.1-5 % by weight.

The oral compositions can be formulated in any suitable application form, such as gels, mouthwashes, toothpowders and toothpastes. They may be formulated into a single formulation or they may be formulated for multi compartment containers into different formulations, e.g. one containing the peroxy bleach and ingredients compatible therewith, and another containing the remaining ingredients.

The oral care compositions of the present invention may furthermore comprise optional, conventional ingredients such as pharmaceutically acceptable carriers like starch, sucrose, water or water/alcohol systems etc.. Small amounts of surfactants may also be included, such as anionic, nonionic and amphoteric surfactants. When formulated into a dentifrice, such formulation may contain all the usual dentifrice ingredients.

Thus, they may comprise particulate abrasive materials such as silicas, aluminas, calcium carbonates, dicalciumphosphates, calcium pyrophosphates hydroxyapatites, trimetaphosphates, insoluble hexametaphosphates and so on, usually in amounts between 5 and 60 % by weight.

Furthermore, the dentifrice formulations may comprise humectants such as glycerol, sorbitol, propyleneglycol, xylitol, lactitol and so on.

Binders and thickeners such as sodium carboxymethylcellulose, xanthan gum, gum arabic etc. may also be included, as well as synthetic polymers such as polyacrylates and carboxyvinyl polymers such as Carbopol®.

Flavours such as peppermint and spearmint oils may also be included, as well as preservatives, opacifying agents, colouring agents, pH-adjusting agents, sweetening agents and so on. Stabilising agents for the organic peroxy acids such as dipicolinic acid or sodium stannate may also be usefully included.

Anti-bacterial agents may also be included such as Triclosan, chlorhexidine, copper-, zinc- and stannous salts such as zinc citrate, sodium zinc citrate and stannous pyrophosphate, sanguinarine extract, metronidazole. Further examples of anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-(4-chloro-6-bromophenol).

Polymeric compounds which can enhance the delivery of active ingredients such as anti-bacterial agents can also be included. Examples of such polymers are copolymers of polyvinylmethylether with maleic anhydride and other similar delivery enhancing polymers, e.g. those described in Furthermore anti-inflammatory agents such as ibuprofen, flurbiprofen, aspirin, indomethacin etc. may also be included.

Anti-caries agents such as sodium- and stannous fluoride, aminefluorides, monosodiumfluorophosphate, casein, plaque buffers such as urea, calcium lactate, calcium glycerophosphate, strontium polyacrylates may also be included. Other optional ingredients include vitamins such as Vitamin C, and plant extracts. Desensitising agents such as potassium citrate, potassium chloride, potasium tartrate, potassium bicarbonate, potassium oxalate, potassium nitrate as well as strontium salts may also be included.

Buffers and salts to buffer the pH and ionic strength of the compositions may also be included. Liposomes and other encapsulates may also be used to improve delivery or stability of active ingredients.

Furthermore, the oral compositions may comprise anti-calculus agents such as alkalimetal pyrophosphates, hypophosphite-containing polymers, organic phosphonates, phosphocitrates etc..

In addition, the compositions may comprise functional biomolecules such as bacteriocins, antibodies, enzymes and so on.

Other optional ingredients that may be included are e.g. bleaching agents, e.g. those described in EP-A-0 545,594, effervescing systems such as sodium bicarbonate/citric acid systems, colour change systems, and so on.

When formulated as a mouthwash, the oral care composition usually comprises a water/alcohol solution, flavour, humectant, sweetener and colorant.

Since the peroxyacids of the invention also have an antimicrobial-property, the composition of the invention are also effective to combat plaque and caries.

The present invention will further be illustrated by way of Example.

### Example I

The bleaching agents were evaluated as follows:
(1) Synthetic hydroxyapatite discs were polished and placed in sterile saliva at 37°C overnight to form a pellicle.
(2) Discs were stained with tea/coffee/iron salts/saliva mixture for seven days at 37°C.
(3) Stained discs were immersed in bleaching solutions for desired time.
(4) The change in colour of the discs was measured using a Minolta chromameter CR-300 in L* a* b* mode. Using L* (initial), L* (soiled), and L* (cleaned), the percentage of stain removed was calculated.

### Example II

### 1. Various peracids were tested according to the method of Example I.

The concentrations of peracids were all 36mM made up in 0.5M sodium bicarbonate.

| Time | (mins) | % stain removed with | | |
|---|---|---|---|---|
| | BIPTA | DPDDA | NAPAA | PAP |
| 0 | 0 | 0 | 0 | 0 |
| 1 | 25 | 16 | 16 | 13 |
| 3 | 39 | 32 | 29 | 28 |
| 5 | 51 | -- | 40 | 39 |

BIPTA = butyl imido peroxytrimellitic acid
DPDDA = 1,10 Diperoxydodecandioic acid (for comparison;
DPDDA is not according to the invention)
NAPAA = monononylamide of peroxyadipic acid
PAP = N-phthalimido hexanoic acid.

## Claims

1. An oral composition with an improved teeth whitening effect, comprising a safe and effective amount of an organic peroxy acid, **characterised in that** the organic peroxy acid is selected from the group consisting of:
cationic peroxycarboxylic acids having the formula 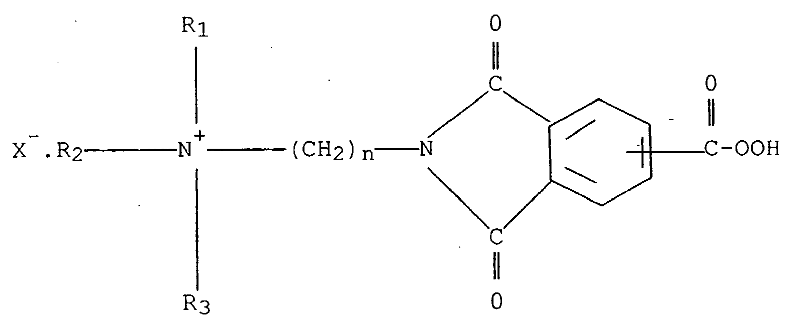
wherein R₁, R₂ and R₃ are each independently a C₁-C₇ alkyl group or C₁-C₇ substituted alkyl group, n is an integer of from 2 to 10 and X is a counter anion.

2. An oral composition with an improved teeth whitening effect, comprising a safe and effective amount of an organic peroxy acid, **characterised in that** the organic peroxy acid is butyl imido peroxytrimellitic acid ("BIPTA").

3. Use of the peroxyacids of claim 1 or 2 as teeth whitening agent in the manufacture of an oral composition having improved teeth whitening properties.

## Patentansprüche

1. Orale Zusammensetzung mit einem verbesserten Zahnweißungseffekt, umfassend eine sichere und wirksame Menge einer organischen Peroxysäure, **dadurch gekennzeichnet, dass** die organische Peroxysäure ausgewählt ist aus der Gruppe, bestehend aus:
kationischen Peroxycarbonsäuren mit der Formel 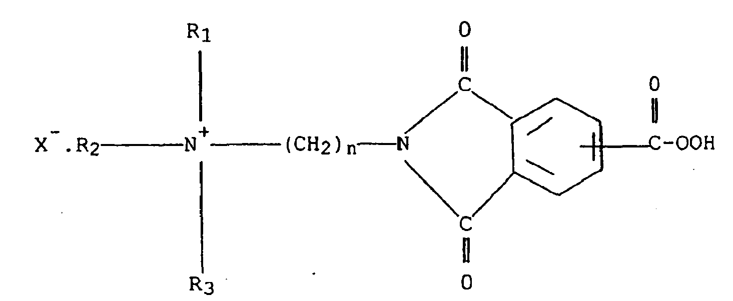
worin R₁, R₂ und R₃ jeweils unabhängig eine C₁-C₇-Alkylgruppe oder substituierte C₁-C₇-Alkylgruppe darstellen, n eine ganze Zahl von 2 bis 10 ist und X ein Gegenanion darstellt.

2. Orale Zusammensetzung mit einem verbesserten Zahnweißungseffekt, umfassend eine sichere und wirksame Menge einer organischen Peroxysäure, **dadurch gekennzeichnet, dass** die organische Peroxysäure Butylimidoperoxytrimellitsäure ("BIPTA") darstellt.

3. Verwendung der Peroxysäuren von Anspruch 1 oder 2 als Zahnweißungsmittel bei der Herstellung einer oralen Zusammensetzung mit verbesserten Zahnweißungseigenschaften.

## Revendications

1. Composition orale présentant un effet amélioré de blanchiment des dents, comprenant une quantité sûre et efficace d'un peroxyacide organique, **caractérisé en ce que** le peroxyacide organique est choisi dans l'ensemble consistant en les acides peroxycarboxyliques cationiques ayant la formule 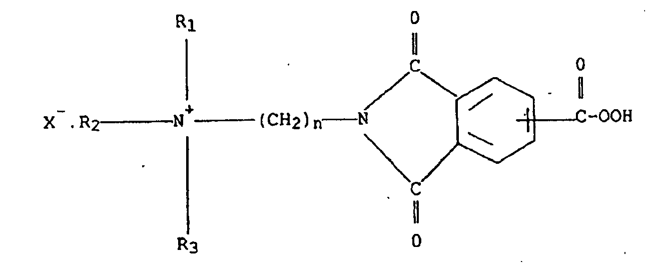 dans laquelle R₁, R₂ et R₃ représentent chacun indépendamment des autres un groupe alkyle en C₁-C₇ ou un groupe alkyle substitué en C₁-C₇, n est un entier de 2 à 10, et X est un contre-anion.

2. Composition orale présentant un effet amélioré de blanchiment des dents, comprenant une quantité sûre et efficace d'un peroxyacide organique, **caractérisée en ce que** le peroxyacide organique est l'acide butylimidoperoxy-trimellitique ("BIPTA").

3. Utilisation des peroxyacides selon la revendication 1 ou 2 en tant qu'agents de blanchiment des dents pour la fabrication d'une composition orale ayant des propriétés améliorées de blanchiment des dents.
